# EUROPEAN PATENT SPECIFICATION

(11) **EP 3 188 057 B1**
(45) Date of publication and mention of the grant of the patent: **29.12.2021**
(21) Application number: 15461589.2
(22) Date of filing: 29.12.2015
(51) Int. Cl.: G16H 40/60, G01N 33/543, G16H 10/40

(54) **DEVICE AND METHOD FOR ELECTRO-CHEMICAL ANALYSIS OF BLOOD THROMBOSIS**
VORRICHTUNG UND VERFAHREN ZUR ELEKTROCHEMISCHEN ANALYSE VON BLUTTHROMBOSE
DISPOSITIF ET PROCÉDÉ D'ANALYSE ÉLECTROCHIMIQUE DE LA THROMBOSE ARTÉRIELLE

(43) Date of publication of application: 05.07.2017
(73) Proprietor: Krawczynski, Tomasz, 02-998 Warszawa (PL); Spralja, Mark, Kensington W8 5JA (GB); Stepien, Piotr, 01-913 Warszawa (PL)
(72) Inventor: Spralja, Mark, Kensington, Greater London W85JA (GB)
(74) Representative: AOMB Polska Sp. z.o.o.

(56) References cited:
- US-A1- 2007 256 943
- US-A1- 2009 131 769
- US-A1- 2009 223 287
- US-A1- 2015 050 678
- US-B2- 8 792 954
- None

## Description

### Technical Field

The invention relates to a device and methodology of Point of Care electro-chemical blood analysis relating to blood thrombosis. More specifically, the invention relates to an electro-chemical blood thrombosis analyser platform and a corresponding method for analysing blood thrombosis realized in such a platform. The invention is applicable to analysing the efficacy of the therapeutic treatment of blood thrombosis (a blood thrombosis analyser) and a methodology for realising more complex Point of Care blood assays.

### Background

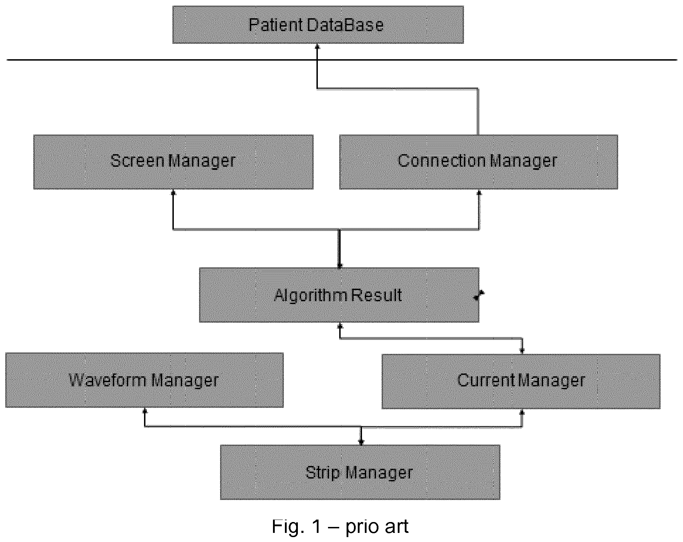

Known blood thrombosis analysers are devices having several functional units, usually all of them integrated in one device (one case), which will be described here with reference to Fig. 1 (prior art). The essential part of any such analyser is a testing unit. This is a unit comprising a sample compartment, i.e. a location/place equipped with means to place or hold therein a chemically treated strip (said strip usually made of paper) on which blood sample to be analysed is deposited (a blood droplet). Further, the testing unit has means for applying a voltage waveform (i.e. a specific voltage as the function of time) to the sample (e.g. electrodes) and another means for reading the electrical response from the sample to said voltage waveform (usually, these are also electrodes, while the electrical response of the sample is e.g. the electrical current as the function of time). The catalyst (specific electrical waveform) is selected such that a chemical reaction is started between the blood sample and strip chemistry, producing an electrical effect (i.e. the progress and/or occurrence of the reaction can be monitored/detected by electrical parameters). Such catalysts are well known in the art - many kinds of strips driven by catalysts are commercially available, e.g. from Roche or Siemens Health Care companies. In addition, the sample compartment is usually equipped with heating means (such as e.g. an electric wire) to heat up the sample and maintain it at required temperature (usually 37°C).

There are also three other important units in the prior art analysers, i.e. the waveform manager, the current manager and the strip manager. The waveform manager is a control unit configured for providing a voltage waveform (i.e. specific voltage as the function of time) to the sample through the dedicated means of the testing unit, as mentioned above. The current manager is configured for reading the electrical response from the sample to said voltage waveform (e.g. the current as the function of time), through the dedicated means of the testing unit, as mentioned above. The strip manager is configured for controlling the conduct of testing, e.g. controlling the presence of the sample in the testing unit and maintaining the sample at the required temperature, through the dedicated means of the testing unit, as mentioned above.

As essential module of the analyser is the algorithm result, which is the unit configured for analysing the electrical response of the sample (provided through the current manager) and calculate the final result of the test.

These four units (the waveform manager, the current manager, the strip manager and the algorithm result) are usually microprocessor-controlled and operable by appropriate software embedded in the analysing device.

The operator (user) can control the whole device and the testing process by means of usually very simple control elements, such as switches, knobs, sliders etc. for controlling some parameters or functions of the device and the testing process. Such control elements can be physical (e.g. a physical electrical switch, a physical knob - potentiometer etc.) or they can be displayed on a touch screen. In any case, the analysers currently known are able to provide only one pre-programmed voltage waveform and apply it to the sample, as well as they can only process and analyse the response from the sample in one pre-programmed way (one algorithm). In the best case, they provide a selection from several pre-programmed voltage waveforms and a selection from several pre-programmed result processing algorithms.

The device may also be equipped with a connection manager (again, a microprocessor-controlled unit) for connecting the analyser to external devices and databases, such as e.g. a patient database, from which test orders may be taken and where test results may be stored. From practical point of view, the presence and possibility to connect to such a patient database is very useful, e.g. for administration of test and their results. However, from the technical point of view, as regards how the blood thrombosis test are performed in the analyser equipped with the testing unit - the patient database is optional.

It should be noted that in the prior art devices the temperature control of the sample (or: in the sample compartment) was realized in an open control mode. Generally speaking, for meaningful blood test results, the chemically treated strip with the blood sample subject to analysis should be kept at the temperature of 37°C (i.e. the temperature of human body) while testing. Overheating of the sample (which could occur e.g. from heating too fast/with too high power) brings a risk of irreversibly damaging the sample. In the prior art devices the sample was heated by a heat wire, without any temperature measurement in the sample compartment. Thus, in order to prevent overheating and destroying of the sample - the heat wire was powered moderately (e.g. at 10% power) and the process of heating up the sample was slow. For measurements in which time is an issue (e.g. accompanying open heart surgery) - special devices were constructed in which blood strips were kept at the proper temperature (e.g. 37°C) ready for testing. In other cases operators and doctors accepted that obtaining test results was time consuming.

Such devices are known are commercially available e.g. from the following companies: Entegrion, CoagMax - Microvisk, Bio-AMD, ACON Laboratories, Inc., CoaguSense, Inc./ Abott; CoaguCheck / Roche, InRatio / Alere, Xprecia Stride / Siemens.

To sum up, the prior art devices work as follows: They are microprocessor-controlled for timing and regulation. Catalytic waveforms (i.e. the voltage waveforms applied to the chemically treated blood strip) are derived from fixed analogue components and have no capacity for modification. The heater applied is a simple Pulse Wave Modulated signal set to a defined duty cycle with no capacity for modification. Samples are collected within the testing unit and the algorithm is also pre-defined with no capacity for modification. When a strip is entered into the unit, the microprocessor starts the process. The heater is started to ensure the blood sample is tested at 37°C. Once the heater is at the correct level or a pre-defined time has elapsed, a voltage waveform is applied to the blood strip. Then samples are collected and entered into the result algorithm, where the final result is calculated.

The major limitations of the prior art devices are the following:
Single voltage catalyst followed by a single unalterable current sense algorithm makes equipment redundant for in-field improvements.

Any field improvements made to the catalytic voltage waveform or the current sensing algorithm cannot be easily incorporated into current blood thrombosis analysers, as it requires re-programming of some units of the device.

Document JP5788874B2 describes a blood analyser that comprises two modules - one with electric sensors and recording means and the other one with analytic means, with communication interface that enables operation of the device, displaying results of the measurements; both modules being in wireless communication.

Document US8792954B2 describes an electronic blood analyser that in its calculation module contains means to send electrical signal which will be used to analyse blood components and means to adjust/calibrate measurement details. See claim 1 in this document for the possibility of programming time intervals at which measurement values are sampled, wherein said programming is effected through a "hand-held receiver in wireless communication with the sensor electronics". This does not provide the versatility of the present inventive solution.

Document JP2015531916A describes a user interface to communicate and analyse the data regarding user's health, interface operable by mobile devices (like smartphones) and connected to the Internet.

Document EP1099114B1 describes blood analyser which has base module and analytic module and interface enabling their communication, said analyser allowing measuring glucose levels or blood clotting. The modular architecture of the device does not provide a possibility to program the voltage/time sequence the sample is subjected to, nor to program the way the results are analysed.

Although selected control or other type sub-units in wireless communication with the rest of the testing device are known, as apparent from the aforementioned citations, nowhere in the prior art it has been proposed to use an external device, such as smartphone, tablet or laptop, in wireless communication with the testing unit, to control the voltage waveform applied to the sample, to analyse the current response from the sample and/or to run the algorithm calculating the final test results. Also, a closed-control loop for controlling the temperature at which the sample is kept, is not known in the prior art.

Document US20150112264A1 describes devices for blood analysis, that comprise mobile interface operable by touchscreen, which can communicate with other components (e.g. modules analysing blood components), said interface being in communication with computer/Internet and enabling updating of settings/software controlling work of said other components. In other words: some control possibilities over medical devices (blood analysers) are delegated to an external unit (interface), but not the possibility to program directly the voltage/time sequence the sample is subjected to, nor to program the way the results are acquired/analysed.

Document US20140304773A1 describes an external wireless interface (such as a smartphone) to remotely control (and in particular: to program!) an implantable medical device. The interface (smartphone) communicates with other, external devices/websites.

Document CA2582775C describes a system for analysing blood components, which utilizes measuring module transmitting gathered data to analytic device, which afterwards processes the data and visualizes the results (in an external wireless "reading module").

### Summary

It is thus the object of the present invention to provide a blood thrombosis analyser overcoming the limitations mentioned above, in particular - being suitable for quick in-field improvements of the waveform applied and the response current analysis algorithm.

It is a further object of the present invention to provide a blood thrombosis analyser capable of controlling the temperature at which the chemically treated strip and sample is kept in the testing unit in a quick, reliable and very precise way, so as to provide fast heating up of the sample and eliminate the risk of sample overheating.

It is still a further object of the present invention to provide a method for analysing blood thrombosis providing the aforementioned advantages and implementable in the inventive analyser.

Actually, the present inventive device and method provide virtually unlimited possibilities of shaping the voltage waveform (voltage as the function of time applied to the sample subject to analysis) as well as possibilities of applying any rules/algorithm for analysing the output signal received from the sample subject to analysis (such as e.g. current vs. time), all this available quickly (within seconds, minutes or hours rather than weeks) through a user interface and/or through changing a corresponding programme in an external device in communication with the testing unit. All this without the need to physically rebuild modules of the analysing apparatus. Also, in-test tuning is very easy to provide within the inventive apparatus and method.

The authors of the present invention unexpectedly noticed that all the aforementioned goals are achievable by the key inventive concept to delegate the waveform manager, current manager, test results analysis and optionally also temperature control functions to an external device (control unit) in wireless communication with the part of the analyzing apparatus where the sample is placed for testing (testing unit) and providing a user interface to or in the control unit. Moreover, the desired advantageous temperature control of the chemically treated strip with the sample in the testing unit is achieved by placing a temperature sensor in the sample compartment of the testing unit, said temperature sensor in communication with the control unit, and controlling the temperature in a closed control loop.

A device for electro-chemical analysis of blood thrombosis, comprising:
a. a testing unit with a sample compartment, equipped with means to place or hold therein a chemically treated strip for a chemical reaction (i.e. a strip that can be driven by a voltage catalyst to induce a chemical reaction) on which a blood sample to be analysed can be deposited, said testing unit further comprising voltage application means (the catalyst), preferably electrodes, for applying a voltage waveform to the sample and response reading means, preferably electrodes, for reading the electrical response from the sample to said voltage waveform;
b. a waveform manager unit configured for providing a voltage waveform to the sample through the voltage application means of the testing unit (the catalyst);
c. a current manager unit configured for reading the electrical response from the sample to said voltage waveform through the response reading means of the testing unit;
d. a strip manager unit configured for controlling the conduct of testing the sample in the sample compartment of the testing;
e. a test result unit configured for analysing the electrical response of the sample provided through the current manager unit and calculate the final result of the test;
according to the invention is characterized in that
the device comprises an external control unit configured for wireless communication with the part of the device comprising the testing unit, while the part of the device which comprises the testing unit is provided with or connected to communication means for wireless communication with the external control unit, wherein said external control unit provides at least the functionality of the waveform manager unit, the current manager unit and the test result unit and provides a user interface enabling the user to program the voltage waveform,wherein the testing unit additionally comprises temperature measurement means, preferably a thermometer, configured for wireless communication with the external control unit and heating means, preferably an electric heater, for heating the sample in the sample compartment,and wherein the device is configured such that a separate wireless communication channel is used for wireless communication between the temperature measurement means, the external control unit and the heating means.

Preferably, the external control unit additionally provides the functionality of the strip manager unit.

Preferably, the user interface of the external control unit enables re-programming of at least one of: the waveform manager unit, the current manager unit, the test result unit and/or the strip manager unit.

Preferably, the user interface of the external control unit enables the user to upload to the waveform manager unit a file containing pairs/series of numbers, said numbers corresponding to voltage values in consecutive moments of time or to voltage values and durations of time intervals over which these voltage values should be applied.

Preferably, the user interface of the external control unit contains input means for inputting pairs/series of values by the user, wherein said input means include any of the following: a keyboard for direct input of numbers, control elements such as sliders, switches, knobs, graphs for adjusting numerical values.

Preferably, the external control unit has a touch screen.

Preferably, the external control unit is a smartphone, a tablet or a laptop.

Preferably, the external control unit is configured to communicate with the part of the device comprising the testing unit by any of the following standards: Bluetooth, WiFi, radio communication.

The invention also includes a method for electro-chemical analysis of blood thrombosis according to claim 10. carried out in a device for analysing blood thrombosis, said device comprising:
a. a testing unit with a sample compartment, equipped with means to place or hold therein a chemically treated strip for a chemical reaction (i.e. a strip that can be driven by a voltage catalyst to induce a chemical reaction) on which a blood sample to be analysed can be deposited, said testing unit further comprising voltage application means, preferably electrodes, for applying a voltage waveform to the sample and response reading means, preferably electrodes, for reading the electrical response from the sample to said voltage waveform;
b. a waveform manager unit configured for providing a voltage waveform to the sample through the voltage application means of the testing unit;
c. a current manager unit configured for reading the electrical response from the sample to said voltage waveform through the response reading means of the testing unit;
d. a strip manager unit configured for controlling the conduct of testing the sample in the sample compartment of the testing;
e. a test result unit configured for analysing the electrical response of the sample provided through the current manager unit to calculate the final result of the test;
the device additionally comprising an external control unit configured for wireless communication with the part of the device comprising the testing unit, while the part of the device which comprises the testing unit is provided with or connected to communication means for wireless communication with the external control unit, wherein said external control unit provides at least the functionality of the waveform manager unit, the current manager unit and the test result unit and provides a user interface enabling the user to program the voltage waveform,
characterized in that
a voltage waveform is programmed by the user through the user interface of the eternal control unit,
a chemically treated strip and a blood sample to be analysed are introduced to the sample compartment of the testing unit, the voltage waveform is applied to the sample as a catalyst to the chemical reaction, an electrical response from the sample to said voltage waveform is read, the electrical response from the sample is analysed by the test result unit and the final test result is calculated.

The inventive method is carried out in a device whose testing unit additionally comprises temperature measurement means, preferably a thermometer, configured for wireless communication with the external control unit and heating means, preferably an electric heater, for heating the sample in the sample compartment and temperature in the sample compartment of the testing unit is controlled at the required 37°C, in a closed-control loop between said temperature measurement means, the external control unit and the heating means.

A separate wireless communication channel is used for wireless communication between the temperature measurement means, the external control unit and the heating means.

Preferably, in the inventive method, in-test tuning is performed by
a. applying an initial voltage waveform to the sample,
b. analysing current feedback from the sample in response to the initial voltage waveform for markers,
c. based on the analysis results, proposing a modified voltage waveform,
d. applying the modified voltage waveform to the sample,
e. taking the current feedback from the sample in response to the modified voltage waveform and feeding it to the test result unit,
f. calculating the test result by the test result unit based on the sample response to the modified voltage waveform.

Preferably, the test results are stored into a patient database or sent out using a communication network, preferably sent to a doctor by e-mail.

Preferably, the external control device is a smartphone, a tablet or a laptop, preferably having a touch screen, and the wireless communication between the external control unit and the part of the device comprising the testing unit is carried out by any of the following standards: Bluetooth, WiFi, radio communication.

In the present invention, separating the voltage waveform from current sense algorithm and makes both of these steps programmable in every axis in compilation time. If a major improvement in the scientific algorithms is made that makes current assays more exact or faster, or if a new assay is invented using the same baseline functions, the inventive solution can be reprogrammed in less than 10 minutes to support it. No analyser or platform on the market can do this and will not be able to keep up with improvements. The inventive solution can support multiple electro-chemical blood assays using each assay's precise requirements. The inventive solution can produce any level of complexity required for the voltage catalyst waveform by storing and synthesizing reprogrammable time based data. According to the inventive solution it is possible to reprogram every element in the entire electro-chemical blood thrombosis testing chain in the field including the final algorithm. The present inventive solution is compatible with all existing (commercially available) chemically treated strips and can use them for testing, dependent on physical dimension constraints.

### Brief Description of the Drawings

Preferred embodiments of the present invention are presented in a more detailed way with reference to the attached drawing, in which:
- Fig. 1: presents a functional scheme and functional modules of a prior art device,

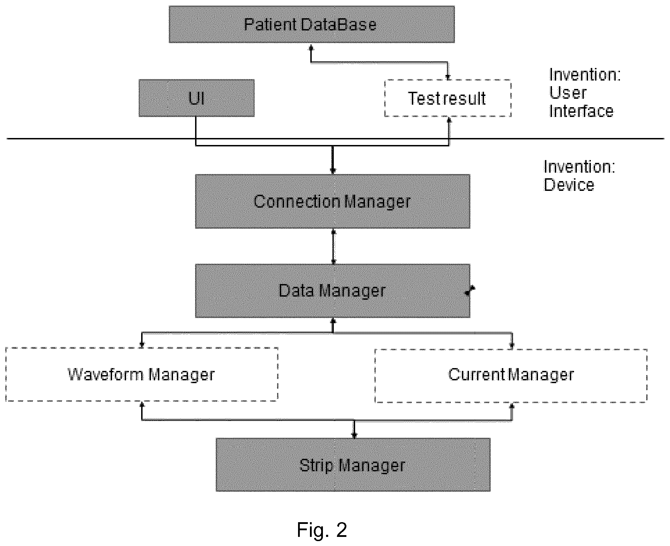

- Fig. 2: shows a functional scheme and functional modules of a device according to the present invention (boxes with dashed frames denote programmable inventive units),
- Figs. 3 and 4: show a typical voltage catalyst (voltage waveform, simple blood strip excitation voltage) and current response (current response which is sampled after a trigger point) - current response (mA) versus time(ms) for sampling (11 - Ixx) obtained with the inventive device, respectively, and
- Fig. 5 (a)-(c): illustrate the ability of the inventive device to program and re-program the voltage waveform: (a) complex blood strip excitation voltage waveform supported by device 10,000 programmable points - voltage (mV) versus time (ms); (b) simple voltage catalyst can be constructed and synthesised in two commands... (c) ...and then rebuilt in another two commands.

### Detailed Description

Preferred embodiments of the invention are described in details below. The examples serve only as an illustration.

It must be noted that although throughout the present specification the invention is described with reference to a blood thrombosis apparatus and method - the key inventive concept to delegate the waveform manager, current manager, test results analysis and optionally also temperature control functions to an external device (control unit) in wireless communication with the part of the analyzing apparatus where the sample is placed for testing (testing unit) is by no means limited to analysis of blood thrombosis. To the contrary, a skilled person would immediately realize the concept is easily and directly (i.e. without need for modifications) applicable to any other type of analysis in which a chemical reaction, started via an electrical impulse (the catalyst), occurring on a chemically treated strip of material is carried out and results of such reaction are detected by observing electrical response of the sample (such as voltage, current etc.) In a broad sense, the concept is applicable to analyzing any chemical reaction, which can produce electrical effect. In a still broader sense, the inventive technique is applicable not just to electro-chemical methods, but to optical, electro-acoustic and many other methods. It is expected that any blood assay could be supported by the inventive technique with the corresponding requisite blood strip. Any blood test, (e.g. toxicological, oncological etc.) that can be miniaturized to the methodology of placing a drop of blood on a chemically treated strip further requiring an electrical catalyst which then provides a known, measurable diagnostic response is a candidate for this invention. It is even more germane where the assay is at a level of complexity as to require pre-analysis to finely adjust input parameters for a more precise result.

### Example 1 - inventive device and method

The inventive device is constructed and works essentially similar to the prior art devices, as described in the introductory part with reference to fig. 1 (prior art), however with differences specified below:
The part of the device which comprises the testing unit is also provided with or connected to communication means for wireless communication with an external control unit. The following functional modules are provided through said external control unit: waveform manager, current manager, algorithm calculating the test results and a user interface. The following functional module - strip manager - may also be provided by the external control unit.

It is to the great benefit of the invention that the waveform manager, the current manager and the test result module are programmable, through the external control unit. The strip manager module may optionally also be programmable.

Programming and re-programming of the aforementioned modules (the waveform manager, the current manager and the test result module) may be realized through uploading appropriate files to the external control unit. In case of the waveform manager, such files may be files with a list of parameters (e.g. to program a particular voltage sequence as the function of time) one can upload to the waveform manager a file (e.g. of txt, csv, xls type etc.) containing pairs/series of numbers, said numbers corresponding to voltage values in consecutive moments of time (measured from a chosen initial moment) or to voltage values and durations of time intervals over which these voltage values should be applied. In one embodiment of the invention, such a text file could contain 10000 pairs of numbers: voltage values from the range from -1V to +1V, more preferably from -1,5V to +1,5V, still more preferably from -2V to +2V, given with time interval of 1ms (millisecond), thus forming a 10 second long voltage waveform in this example. Several times that timespan can be achieved through variation of parameters outside of the text file. Alternatively, the user interface provided by the external control device may contain input means for inputting such pairs of values or a series of values by the user/operator, wherein said input means include, but are not limited to: a keyboard for direct input of numbers, control elements displayed on a touch screen of the external control unit (sliders, switches, knobs, graphs - for adjusting numerical values). In any case, the waveform defined by the waveform manager is in turns applied to the sample in the testing unit. In case of the current manager and the test result module, such files may be program files and contain algorithms for monitoring the electrical response of the sample and for calculating the final test results on the basis of this response and the voltage waveform. Delegating this functionality to an external control unit having a user interface configured for re-programming the waveform manager, the current manager and the test result module provides a flexibility and versatility of voltage waveforms applied and possibilities of analysing results by virtually any method - unheard of in the art.

The external control unit can also provide the functionality of the strip manager module. The progress of the testing process performed for a particular sample can then be monitored and controlled by the user/operator through the user interface provided by the external control unit. This unit can in particular be used to control the temperature in the sample compartment of the testing unit. The same or additional wireless communication channel can be used for temperature control.

In the preferred embodiment of the present invention, the external control unit is an external autonomous programmable device in wireless communication with the testing unit. Preferably, such a device is a smartphone, a tablet or a laptop. The most preferred device is a smartphone, on which appropriate application is running. The most preferred user interface provided by the external control unit is a touch screen with appropriate control elements, as mentioned above.

According to the invention, any suitable wireless communication standards may be applied for communication between the testing unit and the external control unit. Preferred examples of such communication standards include but are not limited to: Bluetooth, WiFi, radio communication, the most preferred being Bluetooth.

The additional feature of the inventive device is a closed-loop temperature control of the compartment of the inventive device where the chemically treated strip and the blood sample are kept for testing (sample compartment). The sample compartment contains a heater or heaters, usually electrical heaters, for heating up the sample. Alternatively such heater(s) may be placed outside the sample compartment, but in the vicinity of the sample, so that to be able to heat the sample compartment. By controlling the electric power provided to the heater(s) - the heat emission (ramping rate) can be controlled. Moreover, in the inventive device, a temperature sensor is placed in the vicinity of the sample for measuring the temperature in the sample compartment. The results of such temperature measurements - according to the invention - are immediately (or in regular time intervals, selected suitably to the sample measurements performed) transmitted by the same or separate wireless channel to the control unit. The control unit, in turns, adjusts the power of the heater(s) heating the sample compartment, so as to maintain the sample at the desired temperature (e.g. 37°C) and prevent overheating, which could destroy the sample. By the closed-control loop it is possible to heat up the sample much faster than in the prior art devices (e.g. by heating at 90% power for 10 seconds rather than at 10% power for over 1 minute) and still save the sample from damage through overheating. Moreover, in the present inventive device and method, it is possible to detect if the sample was destroyed by overheating.

It should be noted that the additional feature related to temperature control described above is completely independent from the voltage control/response current analysis feature, described before. That is to say, the temperature control feature may preferably be, but does not have to be applied together with the voltage/current feature.

### Example 2 - example of blood analysis using the inventive device

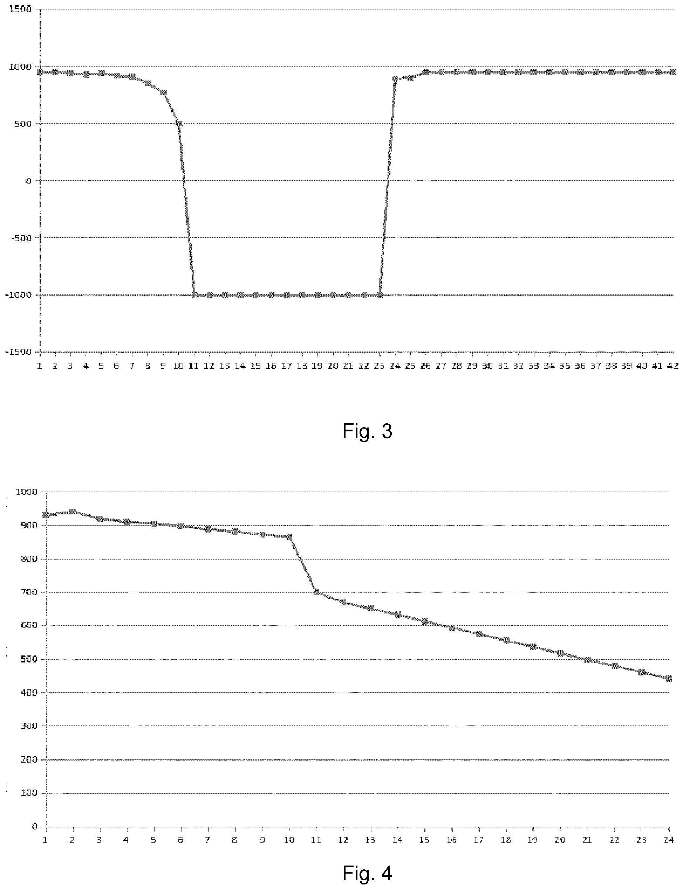

Fig. 3 presents a typical fixed voltage catalyst used in existing electro-chemical devices.
Fig. 4 presents a typical current response of an electro-chemical blood sample over time (current response (mA) versus time(ms) for sampling (11 - Ixx).

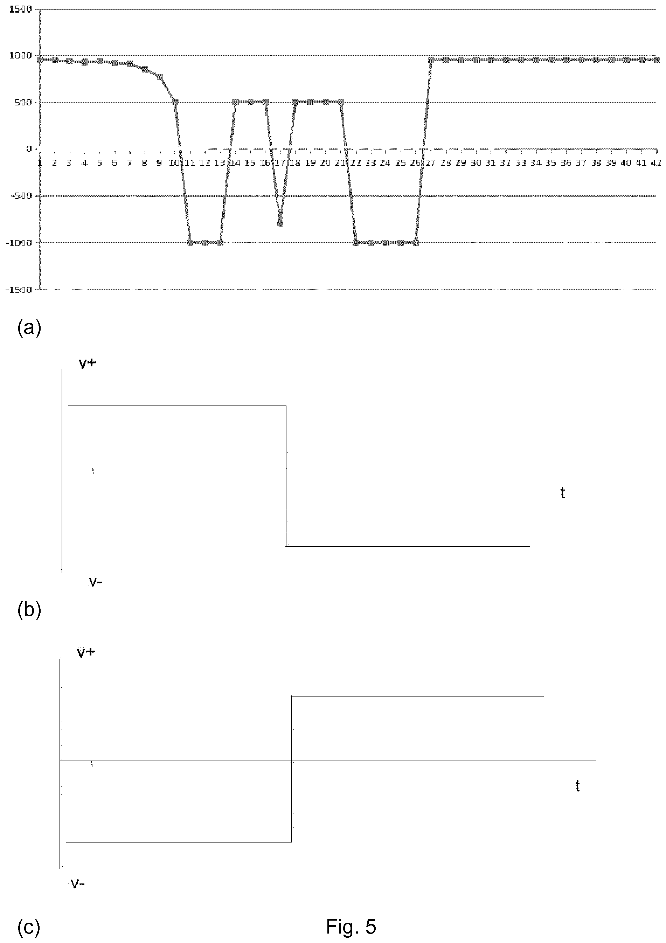

Fig. 5 presents examples of catalytic voltage waveforms the inventive device can program and reprogram in real time, including but not restricted to during the testing phase. Specifically, Fig. 5a: Voltage (mV) versus time (ms).

Test setup:
Positive maximum 1.0 v
Negative minimum -1.0v
Time period for each catalyst (start to finish) 5 seconds,
   An initial voltage catalyst (Figure 5a) is applied to a blood sample that has been placed onto a chemically treated blood strip. A current response represented by Figure 4 is produced by placing a voltage across the catalysed blood sample. Samples (Ix - Ixx) are taken, inserted into an algorithm and a result (r1) is calculated.

From this result (r1), a new voltage catalyst (Figure 5b) or (Figure 5c) is deemed necessary to respond to certain thromboplastic markers thereby optimizing the test and overcoming certain inhibitors. Either of the new voltage catalysts (Figure 5b or 5c) are re-programmed by the inventive device in 2 commands.

Following the reapplication of the new catalyst (Figures 5b or 5c), a 2nd current response, again represented by Figure 4 is produced by placing a voltage across the re-catalyzed blood sample. Samples (Ix - Ixx) are then taken, inserted into a rebased algorithm and a final result (r2) is calculated. The representation of Figure 4 is consistent in terms of a catalyst driven chemical reaction which induces an initially high but unstable current response which then quickly trends to zero over time. The samples are only taken once a consistent declining trend line has been established.

### Example 3 - in-test tuning

As mentioned before, one of the main advantages of the inventive device and method is the re-programmability of the control unit, to be able to use any companies parameters and adjust them as needed.

The future blood assays that the invention can support are predicted to require in-test tuning whereby an initial catalytic-driven analysis produces markers that indicate a modified final electrical catalyst will be required for a more accurate reading for that particular blood type/protein level/etc. The present invention - in particular: the control unit - has this capacity to refine the catalyst in real-time and at any time in the process to complete a far more complex test.

For example, the following in-test tuning scheme can be applied (wherein steps 2, 3 and 5 (in part) are carried out in the control unit):
1. Initial voltage waveform V1 (t) is applied to the blood strip as the catalyst.
2. The current feedback is analyzed for markers.
3. Based on the feedback, a new voltage waveform V2(t) is proposed (created) to be more accurate to the specifics of the blood sample.
4. V2(t) is applied to the blood strip as the new catalyst.
5. Current samples are taken and fed into the analyzing algorithm in the control unit for calculating the final result.

In this way, the technique is applicable not just to electro-chemical methods, but to optical, electro-acoustic and many other methods (examples given above).

Specific attention should be paid to this feature of the invention, so that the re-programmability is noted as being in-test capable.

## Claims

1. A device for electro-chemical analysis of blood thrombosis, comprising:
a. a testing unit with a sample compartment, equipped with means to place or hold therein a chemically treated strip for a chemical reaction on which a blood sample to be analysed can be deposited, said testing unit further comprising voltage application means, preferably electrodes, for applying a voltage waveform to the sample and response reading means, preferably electrodes, for reading the electrical response from the sample to said voltage waveform;
b. a waveform manager unit configured for providing a voltage waveform to the sample through the voltage application means of the testing unit;
c. a current manager unit configured for reading the electrical response from the sample to said voltage waveform through the response reading means of the testing unit;
d. a strip manager unit configured for controlling the conduct of testing the sample in the sample compartment of the testing;
e. a test result unit configured for analysing the electrical response of the sample provided through the current manager unit to calculate the final result of the test;
**characterized in that**
the device comprises an external control unit configured for wireless communication with the part of the device comprising the testing unit, while the part of the device which comprises the testing unit is provided with or connected to communication means for wireless communication with the external control unit, wherein said external control unit provides at least the functionality of the waveform manager unit, the current manager unit and the test result unit and provides a user interface enabling the user to program the voltage waveform,
wherein the testing unit additionally comprises temperature measurement means, preferably a thermometer, configured for wireless communication with the external control unit and heating means, preferably an electric heater, for heating the sample in the sample compartment,
and wherein the device is configured such that a separate wireless communication channel is used for wireless communication between the temperature measurement means, the external control unit and the heating means.

2. The device according to claim 1, **characterized in that** the external control unit additionally provides the functionality of the strip manager unit.

3. The device according to claim 1 or 2, **characterized in that** the user interface of the external control unit enables re-programming of at least one of: the waveform manager unit, the current manager unit, the test result unit and/or the strip manager unit.

4. The device according to claim 1, 2 or 3, **characterized in that** the user interface of the external control unit contains input means for inputting pairs of values by the user, wherein said input means include any of the following: a keyboard for direct input of numbers, control elements such as sliders, switches, knobs, graphs for adjusting numerical values.

5. The device according to claim 1, 2 or 3, **characterized in that** the user interface of the external control unit enables the user to upload to the waveform manager unit a file containing pairs / series of numbers, said numbers corresponding to voltage values in consecutive moments of time or to voltage values and durations of time intervals over which these voltage values should be applied.

6. The device according to claim 1, 2, 3, 4 or 5, **characterized in that** the external control unit has a touch screen.

7. The device according to any one of the claims 1 to 6, **characterized in that** the external control unit is a smartphone, a tablet or a laptop.

8. The device according to any one of the claims 1 to 7, **characterized in that** the external control unit is configured to communicate with the part of the device comprising the testing unit by any of the following standards: Bluetooth, WiFi, radio communication.

9. The device according to any one of the claims 1 to 8, **characterized in that** in-test tuning is performed by
a. applying an initial voltage waveform to the sample,
b. analysing current feedback from the sample in response to the initial voltage waveform for markers,
c. based on the analysis results, proposing a modified voltage waveform,
d. applying the modified voltage waveform to the sample,
e. taking the current feedback from the sample in response to the modified voltage waveform and feeding it to the test result unit,
f. calculating the test result by the test result unit based on the sample response to the modified voltage waveform.

10. A method for electro-chemical analysis of blood thrombosis, carried out in a device for analysing blood thrombosis, said device comprising:
a. a testing unit with a sample compartment, equipped with means to place or hold therein a chemically treated strip for a chemical reaction on which a blood sample to be analysed can be deposited, said testing unit further comprising voltage application means, preferably electrodes, for applying a voltage waveform to the sample and response reading means, preferably electrodes, for reading the electrical response from the sample to said voltage waveform;
b. a waveform manager unit configured for providing a voltage waveform to the sample through the voltage application means of the testing unit;
c. a current manager unit configured for reading the electrical response from the sample to said voltage waveform through the response reading means of the testing unit;
d. a strip manager unit configured for controlling the conduct of testing the sample in the sample compartment of the testing;
e. a test result unit configured for analysing the electrical response of the sample provided through the current manager unit and calculate the final result of the test;
**characterized in that**
the device additionally comprises an external control unit configured for wireless communication with the part of the device comprising the testing unit, while the part of the device which comprises the testing unit is provided with or connected to communication means for wireless communication with the external control unit, wherein said external control unit provides at least the functionality of the waveform manager unit, the current manager unit and the test result unit and provides a user interface enabling the user to program the voltage waveform,
wherein the testing unit additionally comprises temperature measurement means, preferably a thermometer, configured for wireless communication with the external control unit and heating means, preferably an electric heater, for heating the sample in the sample compartment,
and wherein the device is configured such that a separate wireless communication channel is used for wireless communication between the temperature measurement means, the external control unit and the heating means.
a voltage waveform is programmed by the user through the user interface of the eternal control unit,
a chemically treated strip and a blood sample to be analysed are introduced to the sample compartment of the testing unit, the voltage waveform is applied to the sample as a catalyst to the chemical reaction, an electrical response from the sample to said voltage waveform is read, the electrical response from the sample is analysed by the test result unit and the final test result is calculated.

11. The method according to claim 10, **characterized in that** it is carried out in a device whose testing unit additionally comprises temperature measurement means, preferably a thermometer, configured for wireless communication with the external control unit and heating means, preferably an electric heater, for heating the sample in the sample compartment and temperature in the sample compartment of the testing unit is controlled at the required 37°C, in a closed-control loop between said temperature measurement means, the external control unit and the heating means.

12. The method according to claim 10 or 11, **characterized in that** in-test tuning is performed by
a. applying an initial voltage waveform to the sample,
b. analysing current feedback from the sample in response to the initial voltage waveform for markers,
c. based on the analysis results, proposing a modified voltage waveform,
d. applying the modified voltage waveform to the sample,
e. taking the current feedback from the sample in response to the modified voltage waveform and feeding it to the test result unit,
f. calculating the test result by the test result unit based on the sample response to the modified voltage waveform.

13. The method according to any one of the claims from 10 to 12, **characterized in that** the test results are stored into a patient database or sent out using a communication network, preferably sent to a doctor by e-mail.

14. The method according to any one of the claims from 10 to 13, **characterized in that** the external control device is a smartphone, a tablet or a laptop, preferably having a touch screen, and the wireless communication between the external control unit and the part of the device comprising the testing unit is carried out by any of the following standards: Bluetooth, WiFi, radio communication.

## Patentansprüche

1. Vorrichtung zur elektrochemischen Analyse von Blutthrombose, umfassend:
a. eine Testeinheit mit einer Probenkammer, die mit Mitteln ausgestattet ist, um darin einen chemisch behandelten Streifen für eine chemische Reaktion zu platzieren oder zu halten, auf den eine zu analysierende Blutprobe aufgebracht werden kann, wobei die Testeinheit ferner Mittel zum Anlegen einer Spannung, vorzugsweise Elektroden, zum Anlegen einer Spannungswellenform an die Probe und Mittel zum Lesen der Antwort, vorzugsweise Elektroden, zum Lesen der elektrischen Antwort von der Probe auf die Spannungswellenform umfasst;
b. eine Wellenformmanagereinheit, die so konfiguriert ist, dass sie der Probe über die Spannungsanlegeeinrichtung der Testeinheit eine Spannungswellenform zuführt;
c. eine Stromverwaltungseinheit, die so konfiguriert ist, dass sie die elektrische Antwort der Probe auf die Spannungswellenform über die Antwortlesemittel der Testeinheit liest;
d. eine Streifenverwaltungseinheit, die so konfiguriert ist, dass sie die Durchführung des Tests der Probe in der Probenkammer des Tests steuert;
e. eine Testergebniseinheit, die so konfiguriert ist, dass sie die elektrische Reaktion der durch die Strommanagereinheit bereitgestellten Probe analysiert, um das Endergebnis des Tests zu berechnen;
**dadurch gekennzeichnet, dass**
die Vorrichtung eine externe Steuereinheit umfasst, die für eine drahtlose Kommunikation mit dem Teil der Vorrichtung konfiguriert ist, der die Testeinheit umfasst, während der Teil der Vorrichtung, der die Testeinheit umfasst, mit Kommunikationsmitteln für eine drahtlose Kommunikation mit der externen Steuereinheit versehen oder mit diesen verbunden ist, wobei die externe Steuereinheit zumindest die Funktionalität der Wellenformmanagereinheit, der Strommanagereinheit und der Testergebniseinheit bereitstellt und eine Benutzerschnittstelle bereitstellt, die es dem Benutzer ermöglicht, die Spannungswellenform zu programmieren,
wobei die Testeinheit zusätzlich Temperaturmessmittel, vorzugsweise ein Thermometer, das für eine drahtlose Kommunikation mit der externen Steuereinheit konfiguriert ist, und Heizmittel, vorzugsweise eine elektrische Heizung, zum Erwärmen der Probe in der Probenkammer umfasst,
und wobei die Vorrichtung so konfiguriert ist, dass ein separater drahtloser Kommunikationskanal für die drahtlose Kommunikation zwischen dem Temperaturmessmittel, der externen Steuereinheit und dem Heizmittel verwendet wird.

2. Vorrichtung nach Anspruch 1, **dadurch gekennzeichnet, dass** die externe Steuereinheit zusätzlich die Funktionalität der Streifenverwaltungseinheit bereitstellt.

3. Vorrichtung nach Anspruch 1 oder 2, **dadurch gekennzeichnet, dass** die Benutzerschnittstelle der externen Steuereinheit die Neuprogrammierung von mindestens einer der folgenden Einheiten ermöglicht: der Wellenformmanagereinheit, der Stromverwaltungseinheit, der Testergebniseinheit und/oder der Streifenverwaltungseinheit.

4. Vorrichtung nach Anspruch 1, 2 oder 3, **dadurch gekennzeichnet, dass** die Benutzerschnittstelle der externen Steuereinheit Eingabemittel zur Eingabe von Wertepaaren durch den Benutzer enthält, wobei die Eingabemittel eines der folgenden umfassen: eine Tastatur zur direkten Eingabe von Zahlen, Steuerelemente wie Schieberegler, Schalter, Knöpfe, Graphen zur Einstellen numerischer Werte.

5. Vorrichtung nach Anspruch 1, 2 oder 3, **dadurch gekennzeichnet, dass** die Benutzerschnittstelle der externen Steuereinheit es dem Benutzer ermöglicht, in die Wellenformmanagereinheit eine Datei hochzuladen, die Zahlenpaare/-reihen enthält, wobei die Zahlen Spannungswerten in aufeinanderfolgenden Zeitpunkten oder Spannungswerten und Dauern von Zeitintervallen entsprechen, über die diese Spannungswerte angelegt werden sollten.

6. Vorrichtung nach Anspruch 1, 2, 3, 4 oder 5, **dadurch gekennzeichnet, dass** die externe Steuereinheit einen Touchscreen aufweist.

7. Vorrichtung nach einem der Ansprüche 1 bis 6, **dadurch gekennzeichnet, dass** die externe Steuereinheit ein Smartphone, ein Tablet oder ein Laptop ist.

8. Vorrichtung nach einem der Ansprüche 1 bis 7, **dadurch gekennzeichnet, dass** die externe Steuereinheit so konfiguriert ist, dass sie mit dem Teil der Vorrichtung, der die Testeinheit umfasst, über einen der folgenden Standards kommuniziert: Bluetooth, WLAN, Funkkommunikation.

9. Vorrichtung nach einem der Ansprüche 1 bis 8, **dadurch gekennzeichnet, dass** die Abstimmung im Test durch Folgendes erfolgt:
a. Anlegen einer anfänglichen Spannungswellenform an die Probe,
b. Analyse der Stromrückmeldung von der Probe als Reaktion auf die anfängliche Spannungswellenform für Marker,
c. Vorschlag einer geänderten Spannungswellenform auf der Grundlage der Analyseergebnisse,
d. Anlegen der modifizierten Spannungswellenform an die Probe,
e. Entnahme der Stromrückmeldung von der Probe als Reaktion auf die modifizierte Spannungswellenform und Weiterleitung an die Testergebniseinheit,
f. Berechnung des Testergebnisse durch die Testergebniseinheit auf der Grundlage der Reaktion der Probe auf die modifizierte Spannungswellenform.

10. Verfahren zur elektrochemischen Analyse von Blutthrombose, das in einer Vorrichtung zur Analyse von Blutthrombose durchgeführt wird, wobei die Vorrichtung umfasst:
a. eine Testeinheit mit einer Probenkammer, die mit Mitteln ausgestattet ist, um darin einen chemisch behandelten Streifen für eine chemische Reaktion zu platzieren oder zu halten, auf den eine zu analysierende Blutprobe aufgebracht werden kann, wobei die Testeinheit ferner Mittel zum Anlegen einer Spannung, vorzugsweise Elektroden, zum Anlegen einer Spannungswellenform an die Probe und Mittel zum Lesen der Antwort, vorzugsweise Elektroden, zum Lesen der elektrischen Antwort von der Probe auf die Spannungswellenform umfasst;
b. eine Wellenformmanagereinheit, die so konfiguriert ist, dass sie der Probe über die Spannungsanlegeeinrichtung der Testeinheit eine Spannungswellenform zuführt;
c. eine Stromverwaltungseinheit, die so konfiguriert ist, dass sie die elektrische Antwort der Probe auf die Spannungswellenform über die Antwortlesemittel der Testeinheit liest;
d. eine Streifenverwaltungseinheit, die so konfiguriert ist, dass sie die Durchführung des Tests der Probe in der Probenkammer des Tests steuert;
e. eine Testergebniseinheit, die so konfiguriert ist, dass sie die elektrische Reaktion der durch die Strommanagereinheit bereitgestellten Probe analysiert und das Endergebnis des Tests berechnet;
**dadurch gekennzeichnet, dass**
die Vorrichtung zusätzlich eine externe Steuereinheit umfasst, die für eine drahtlose Kommunikation mit dem Teil der Vorrichtung konfiguriert ist, der die Testeinheit umfasst, während der Teil der Vorrichtung, der die Testeinheit umfasst, mit Kommunikationsmitteln für eine drahtlose Kommunikation mit der externen Steuereinheit versehen oder daran angeschlossen ist, wobei die externe Steuereinheit zumindest die Funktionalität der Wellenformmanagereinheit, der Stromverwaltungseinheit und der Testergebniseinheit bereitstellt und eine Benutzerschnittstelle bereitstellt, die es dem Benutzer ermöglicht, die Spannungswellenform zu programmieren,
wobei die Testeinheit zusätzlich Temperaturmessmittel, vorzugsweise ein Thermometer, das für eine drahtlose Kommunikation mit der externen Steuereinheit konfiguriert ist, und Heizmittel, vorzugsweise ein elektrisches Heizgerät, zum Erwärmen der Probe in dem Probenkammer umfasst,
und wobei die Vorrichtung so konfiguriert ist, dass ein separater drahtloser Kommunikationskanal für die drahtlose Kommunikation zwischen dem Temperaturmessmittel, der externen Steuereinheit und dem Heizmittel verwendet wird,
eine Spannungswellenform durch den Benutzer über die Benutzerschnittstelle der externen Steuereinheit programmiert wird,
ein chemisch behandelter Streifen und eine zu analysierende Blutprobe in das Probenkammer der Testeinheit eingeführt werden, die Spannungswellenform an die Probe als Katalysator für die chemische Reaktion angelegt wird, eine elektrische Antwort der Probe auf die Spannungswellenform gelesen wird, die elektrische Antwort der Probe durch die Testergebniseinheit analysiert wird und das endgültige Testergebnis berechnet wird.

11. Verfahren nach Anspruch 10, **dadurch gekennzeichnet, dass** es in einer Vorrichtung durchgeführt wird, deren Testeinheit zusätzlich eine Temperaturmessmittel, vorzugsweise ein Thermometer, die für eine drahtlose Kommunikation mit der externen Steuereinheit konfiguriert ist, und Heizmittel, vorzugsweise eine elektrische Heizgerät, zum Erwärmen der Probe in der Probenkammer umfasst, und dass die Temperatur in der Probenkammer der Testeinheit in einem geschlossenen Regelkreis zwischen der Temperaturmessmittel, der externen Steuereinheit und der Heizmittel auf die erforderlichen 37 °C geregelt wird.

12. Verfahren nach Anspruch 10 oder 11, **dadurch gekennzeichnet, dass die** Testabstimmung im Test durch Folgendes erfolgt:
a. Anlegen einer anfänglichen Spannungswellenform an die Probe,
b. Analyse der Stromrückmeldung von der Probe als Reaktion auf die anfängliche Spannungswellenform für Marker,
c. Vorschlag einer geänderten Spannungswellenform auf der Grundlage der Analyseergebnisse,
d. Anlegen der modifizierten Spannungswellenform an die Probe,
e. Entnahme der Stromrückmeldung von der Probe als Reaktion auf die modifizierte Spannungswellenform und Weiterleitung an die Testergebniseinheit,
f. Berechnung des Testergebnisse durch die Testergebniseinheit auf der Grundlage der Reaktion der Probe auf die modifizierte Spannungswellenform.

13. Verfahren nach einem der Ansprüche 10 bis 12, **dadurch gekennzeichnet, dass** die Testergebnisse in einer Patientendatenbank gespeichert oder über ein Kommunikationsnetz, vorzugsweise per E-Mail, an einen Arzt versandt werden.

14. Verfahren nach einem der Ansprüche 10 bis 13, **dadurch gekennzeichnet, dass** es sich bei der externen Steuereinheit um ein Smartphone, ein Tablet oder einen Laptop, vorzugsweise mit Touchscreen, handelt und die drahtlose Kommunikation zwischen dem externen Steuereinheit und dem Teil der Vorrichtung, der die Testeinheit umfasst, nach einem der folgenden Standards erfolgt: Bluetooth, WLAN, Funkkommunikation.

## Revendications

1. Dispositif d'analyse électrochimique de la thrombose du sang, comprenant :
a. une unité de test avec un compartiment à échantillon, équipé de moyens pour placer ou maintenir dans celui-ci une bande traitée chimiquement pour une réaction chimique sur laquelle un échantillon de sang à analyser peut être déposé, ladite unité de test comprenant en outre des moyens d'application de tension, de préférence des électrodes, pour appliquer une forme d'onde de tension à l'échantillon et des moyens de lecture de réponse, de préférence des électrodes, pour lire la réponse électrique de l'échantillon à ladite forme d'onde de tension ;
b. une unité de gestion de forme d'onde configurée pour fournir une forme d'onde de tension à l'échantillon par le biais du moyen d'application de tension de l'unité de test ;
c. une unité de gestion de courant configurée pour lire la réponse électrique de l'échantillon à ladite forme d'onde de tension par l'intermédiaire du moyen de lecture de réponse de l'unité de test ;
d. une unité de gestion de bande configurée pour commander la conduite de l'essai de l'échantillon dans le compartiment d'échantillon de l'essai ;
e. une unité de résultat de test configurée pour analyser la réponse électrique de l'échantillon fournie par l'unité de gestion du courant pour calculer le résultat final du test ;
**caractérisé en ce que**
le dispositif comprend une unité de commande externe configurée pour une communication sans fil avec la partie du dispositif comprenant l'unité de test, tandis que la partie du dispositif qui comprend l'unité de test est pourvue de ou connectée à des moyens de communication pour une communication sans fil avec l'unité de commande externe, dans lequel ladite unité de commande externe fournit au moins la fonctionnalité de l'unité de gestion de forme d'onde, l'unité de gestion de courant et l'unité de résultat de test et fournit une interface utilisateur permettant à l'utilisateur de programmer la forme d'onde de tension,
dans lequel l'unité de test comprend en outre des moyens de mesure de la température, de préférence un thermomètre, configurés pour une communication sans fil avec l'unité de commande externe et des moyens de chauffage, de préférence un chauffage électrique, pour chauffer l'échantillon dans le compartiment à échantillon, et dans lequel le dispositif est configuré de telle sorte qu'un canal de communication sans fil séparé est utilisé pour la communication sans fil entre le moyen de mesure de la température, l'unité de commande externe et le moyen de chauffage.

2. Dispositif selon la revendication 1, **caractérisé en ce que** l'unité de commande externe assure en outre la fonctionnalité de l'unité de gestion des bandes.

3. Dispositif selon la revendication 1 ou 2, **caractérisé en ce que** l'interface d'utilisateur de l'unité de commande externe permet de reprogrammer au moins l'un des éléments suivants : l'unité de gestion de forme d'onde, l'unité de gestion de courant, l'unité de résultat de test et/ou l'unité de gestion de bande.

4. Dispositif selon la revendication 1, 2 ou 3, **caractérisé en ce que** l'interface d'utilisateur de l'unité de commande externe contient des moyens d'entrée pour l'entrée de paires de valeurs par l'utilisateur, dans lequel lesdits moyens d'entrée comprennent l'un des éléments suivants : un clavier pour la saisie directe de chiffres, des éléments de commande tels que des curseurs, des commutateurs, des boutons, des graphiques pour le réglage des valeurs numériques.

5. Dispositif selon la revendication 1, 2 ou 3, **caractérisé en ce que** l'interface d'utilisateur de l'unité de commande externe permet à l'utilisateur de télécharger vers l'unité de gestion de formes d'onde un fichier contenant des paires/séries de nombres, lesdits nombres correspondant à des valeurs de tension à des moments consécutifs du temps ou à des valeurs de tension et des durées d'intervalles de temps sur lesquels ces valeurs de tension doivent être appliquées.

6. Dispositif selon la revendication 1, 2, 3, 4 or 5, **caractérisé en ce que** l'unité de commande externe est dotée d'un écran tactile.

7. Dispositif selon l'une quelconque des revendications 1 à 6, **caractérisé en ce que** l'unité de commande externe est un smartphone, une tablette ou un ordinateur portable.

8. Dispositif selon l'une quelconque des revendications 1 à 7, **caractérisé en ce que** l'unité de commande externe est configurée pour communiquer avec la partie du dispositif comprenant l'unité de test par l'une des normes suivantes : Bluetooth, WiFi, communication radio.

9. Dispositif selon l'une quelconque des revendications 1 à 8, **caractérisé en ce que** réglage en cours de test est effectué
a. en appliquant une forme d'onde de tension initiale à l'échantillon,
b. en analysant le retour de courant de l'échantillon en réponse à la forme d'onde de tension initiale pour les marqueurs,
c. sur la base des résultats de l'analyse, en proposant une forme d'onde de tension modifiée,
d. en appliquant la forme d'onde de tension modifiée à l'échantillon,
e. en prenant le retour de courant de l'échantillon en réponse à la forme d'onde de tension modifiée et en l'envoyant à l'unité de résultats de test,
f. en calculant le résultat du test par l'unité de résultat du test sur la base de la réponse de l'échantillon à la forme d'onde de tension modifiée.

10. Procédé d'analyse électrochimique de la thrombose sanguine, mis en oeuvre dans un dispositif d'analyse de la thrombose sanguine, ledit dispositif comprenant :
a. une unité de test avec un compartiment à échantillon, équipé de moyens pour placer ou maintenir dans celui-ci une bande traitée chimiquement pour une réaction chimique sur laquelle un échantillon de sang à analyser peut être déposé, ladite unité de test comprenant en outre des moyens d'application de tension, de préférence des électrodes, pour appliquer une forme d'onde de tension à l'échantillon et des moyens de lecture de réponse, de préférence des électrodes, pour lire la réponse électrique de l'échantillon à ladite forme d'onde de tension ;
b. une unité de gestion de forme d'onde configurée pour fournir une forme d'onde de tension à l'échantillon par le biais du moyen d'application de tension de l'unité de test ;
c. une unité de gestion de courant configurée pour lire la réponse électrique de l'échantillon à ladite forme d'onde de tension par l'intermédiaire du moyen de lecture de réponse de l'unité de test ;
d. une unité de gestion de bande configurée pour commander la conduite de l'essai de l'échantillon dans le compartiment d'échantillon de l'essai ;
e. une unité de résultat de test configurée pour analyser la réponse électrique de l'échantillon fournie par l'unité de gestion du courant et calculer le résultat final du test ;
**caractérisé en ce que**
le dispositif comprend en outre une unité de commande externe configurée pour une communication sans fil avec la partie du dispositif comprenant l'unité de test, tandis que la partie du dispositif qui comprend l'unité de test est pourvue de ou connectée à des moyens de communication pour une communication sans fil avec l'unité de commande externe, dans lequel ladite unité de commande externe fournit au moins la fonctionnalité de l'unité de gestion de forme d'onde, l'unité de gestion de courant et l'unité de résultat de test et fournit une interface utilisateur permettant à l'utilisateur de programmer la forme d'onde de tension,
dans lequel l'unité de test comprend en outre des moyens de mesure de la température, de préférence un thermomètre, configurés pour une communication sans fil avec l'unité de commande externe et des moyens de chauffage, de préférence un chauffage électrique, pour chauffer l'échantillon dans le compartiment à échantillon,
et dans lequel le dispositif est configuré de telle sorte qu'un canal de communication sans fil séparé est utilisé pour la communication sans fil entre le moyen de mesure de la température, l'unité de commande externe et le moyen de chauffage.
une forme d'onde de tension est programmée par l'utilisateur à travers l'interface d'utilisateur de l'unité de commande externe,
une bandelette traitée chimiquement et un échantillon de sang à analyser sont introduits dans le compartiment à échantillons de l'unité de test, la forme d'onde de tension est appliquée à l'échantillon comme catalyseur de la réaction chimique, une réponse électrique de l'échantillon à ladite forme d'onde de tension est lue, la réponse électrique de l'échantillon est analysée par l'unité de résultat de test et le résultat final du test est calculé.

11. Procédé selon la revendication 10, **caractérisé en ce que** elle est réalisée dans un dispositif
dont l'unité d'essai comprend en outre des moyens de mesure de la température, de préférence un thermomètre, configurés pour une communication sans fil avec l'unité de commande externe et des moyens de chauffage, de préférence un chauffage électrique, pour chauffer l'échantillon dans le compartiment à échantillon et la température dans le compartiment à échantillon de l'unité d'essai est contrôlée à la température requise de 37°C, dans une boucle de commande fermée entre lesdits moyens de mesure de la température, l'unité de commande externe et les moyens de chauffage.

12. Procédé selon la revendication 10 ou 11, **caractérisé en ce que** réglage en cours de test est effectué
a. en appliquant une forme d'onde de tension initiale à l'échantillon,
b. en analysant le retour de courant de l'échantillon en réponse à la forme d'onde de tension initiale pour les marqueurs,
c. sur la base des résultats de l'analyse, en proposant une forme d'onde de tension modifiée,
d. en appliquant la forme d'onde de tension modifiée à l'échantillon,
e. en prenant le retour de courant de l'échantillon en réponse à la forme d'onde de tension modifiée et en l'envoyant à l'unité de résultats de test,
f. en calculant le résultat du test par l'unité de résultat du test sur la base de la réponse de l'échantillon à la forme d'onde de tension modifiée.

13. Procédé selon l'une quelconque des revendications 10 à12, **caractérisé en ce que**
les résultats des tests sont stockés dans une base de données du patient ou envoyés par un réseau de communication, de préférence envoyés à un médecin par courrier électronique.

14. Procédé selon l'une quelconque des revendications 10 à13, **caractérisé en ce que**
le dispositif de commande externe est un smartphone, une tablette ou un ordinateur portable, ayant de préférence un écran tactile, et la communication sans fil entre l'unité de commande externe et la partie du dispositif comprenant l'unité de test est effectuée par l'une des normes suivantes : Bluetooth, WiFi, communication radio.
